(19) Europäisches Patentamt European Patent Office Office européen des brevets

(11) EP 4 378 376 A1

(12) EUROPEAN PATENT APPLICATION
published in accordance with Art. 153(4) EPC

(43) Date of publication:
05.06.2024 Bulletin 2024/23

(21) Application number: 22849396.1

(22) Date of filing: 22.07.2022

(51) International Patent Classification (IPC):
A61B 5/00 (2006.01)     A61B 5/11 (2006.01)
A61B 5/113 (2006.01)

(52) Cooperative Patent Classification (CPC):
A61B 5/00; A61B 5/11; A61B 5/113

(86) International application number:
PCT/JP2022/028495

(87) International publication number:
WO 2023/008334 (02.02.2023 Gazette 2023/05)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 28.07.2021 JP 2021123243

(71) Applicant: Minebea Mitsumi Inc.
Kitasaku-gun, Nagano 3890293 (JP)

(72) Inventors:
• SUGAWARA, Satoshi
  Kitasaku-gun, Nagano 389-0293 (JP)
• IIDA, Norihito
  Kitasaku-gun, Nagano 389-0293 (JP)
• TAKAHASHI, Hideyuki
  Kitasaku-gun, Nagano 389-0293 (JP)

(74) Representative: Zabel, Julia Elisabeth
Einsel Attorney at Law und
Rechtsanwälte PartmbB
Große Bäckerstraße 3
20095 Hamburg (DE)

(54) **BIOLOGICAL INFORMATION DISPLAY SYSTEM**

(57) According to the present invention, a biological information display system (100) for displaying biological information of a subject on a bed (BD) includes: a biological signal acquisition unit (1) that acquires a biological signal of the subject; a biological information acquisition unit (31) that acquires biological information of the subject on the basis of the biological signal; and a display control unit (32) that displays the biological information on a display unit (5). In a period in which the biological information acquisition unit is not capable of acquiring the biological information, the display control unit displays past information that is the biological information acquired prior to the period, along with time information indicating a period in which the past information was acquired, on the display unit.

FIG. 1

EP 4 378 376 A1

## Description

Technical Field

The present invention relates to a biological information display system.

Background Art

In the field of medical care and nursing care, a system has been proposed to acquire biological information of a subject on a bed without attaching a measuring device to the body of the subject. Specifically, for example, a known system detects a load of a subject on a bed through a load detector and calculates a respiration rate and a heart rate of the subject based on the detected load.

In such a system, it is not always possible to acquire accurate biological information. In this regard, Patent Document 1 discloses a biological information output device. The biological information output device does not output biological information determined to be unreliable, or outputs the biological information together with identification information indicating unreliability. According to Patent Document 1, a user of the biological information can use only highly reliable biological information or can use biological information according to the reliability (Patent Document 1, paragraphs 0012 to 0013).

Citation List

Patent Literature

Patent Document 1: JP 6697985 B

Summary of Invention

Technical Problem

However, in the field of medical care and nursing care, a system with a short period of interruption in the display of biological information is desired. On the other hand, the utility value of unreliable information, even though displayed, is small in the field of medical care and nursing care.

An object of the present invention is to provide a biological information display system capable of displaying useful biological information to a user even during a period when accurate biological information cannot be acquired.

Solution to Problem

According to one aspect of the present invention,
a biological information display system for displaying biological information of a subject on a bed is provided.

The biological information display system includes:

a biological signal acquisition unit configured to acquire a biological signal of the subject;
a biological information acquisition unit configured to acquire the biological information of the subject based on the biological signal; and
a display control unit configured to display the biological information at a display unit.

During a period when the biological information acquisition unit cannot acquire the biological information, the display control unit displays past information being the biological information acquired before the period at the display unit together with time information indicating a time when the past information is acquired.

Advantageous Effects of Invention

The biological information display system of the present invention can display useful biological information to a user even during a period when accurate biological information cannot be acquired.

Brief Description of Drawings

FIG. 1 is a block diagram illustrating a configuration of a biological information display system.
FIG. 2 is an explanatory diagram illustrating an arrangement of a load detector with respect to a bed.
FIG. 3 is a flowchart illustrating a method of displaying biological information using the biological information display system.
FIG. 4(a) is an explanatory diagram conceptually illustrating how the centroid of a subject vibrates in the body axis direction of the subject according to respiration of the subject. FIG. 4(b) is a graph illustrating an example of a respiration waveform drawn based on the vibration of the centroid of the subject according to the respiration of the subject.
FIG. 5(a) is an explanatory diagram illustrating how a present respiration rate (present information) is displayed at a display unit. FIG. 5(b) is an explanatory diagram illustrating how a past respiration rate (past information) and time information are displayed at the display unit.
FIG. 6 is an explanatory diagram illustrating how the contents of information displayed at the display unit fluctuate according to the control of a display control unit.

Description of Embodiments

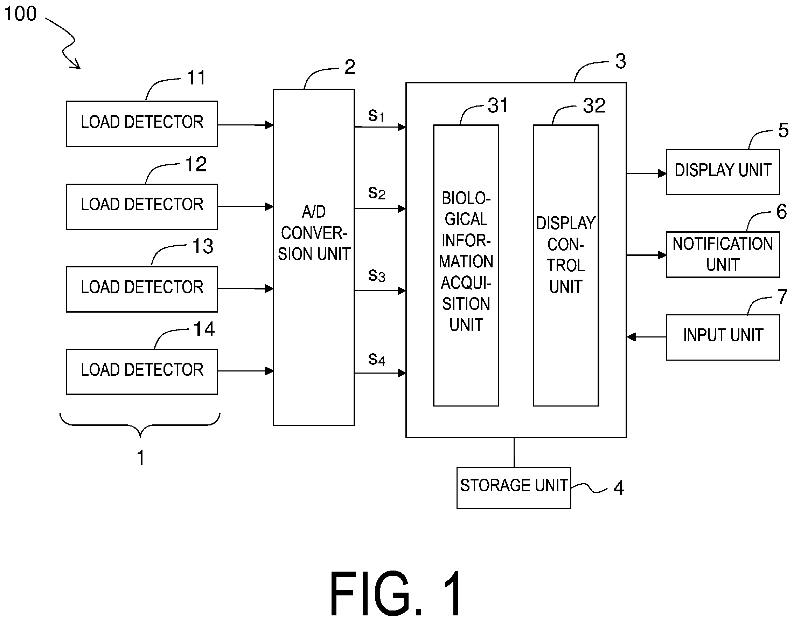

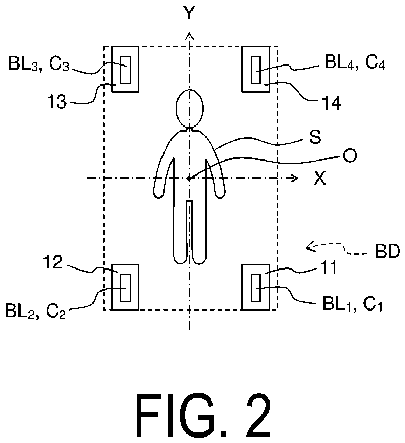

A biological information display system 100 (FIG. 1) of an embodiment of the present invention will now be described by way of an example when used together with a bed BD (FIG. 2) to display the respiration rate of a subject S on the bed BD.

As illustrated in FIG. 1, the biological information display system 100 of the present embodiment mainly includes a load detection unit (biological signal acquisition unit) 1, a control unit 3, a storage unit 4, and a display unit 5. A load detection unit 1 and the control unit 3 are connected via an A/D conversion unit 2. A notification unit 6 and an input unit 7 are further connected to the control unit 3.

The load detection unit 1 includes four load detectors 11, 12, 13, and 14. Each of the load detectors 11, 12, 13, and 14 is a load detector for detecting a load by using, for example, a beam type load cell. Such a load detector is described, for example, in JP 4829020 B and JP 4002905 B. The load detectors 11, 12, 13 and 14 are connected to the A/D conversion unit 2 by wiring or radio, respectively.

As illustrated in FIG. 2, the four load detectors 11 to 14 of the load detection unit 1 are respectively located under casters $C_1$, $C_2$, $C_3$, and $C_4$ attached to the lower end parts of legs $BL_1$, $BL_2$, $BL_3$, and $BL_4$ at the four corners of the bed BD used by the subject S.

The A/D conversion unit 2 includes an A/D converter for converting an analog signal from the load detection unit 1 into a digital signal, and is connected to each of the load detection unit 1 and the control unit 3 by wired or wirelessly.

The control unit 3 is a dedicated or general-purpose computer, and a biological information acquisition unit 31 and a display control unit 32 are constructed inside.

The storage unit 4 is a storage device for storing data used in the biological information display system 100, and, for example, a hard disk (magnetic disk) can be used.

The display unit 5 is a monitor, such as a liquid crystal monitor, for displaying the biological information of the subject acquired by the biological information acquisition unit 31 to the user of the biological information display system 100 based on an instruction of the display control unit 32.

The notification unit 6 includes a device, for example, a speaker, for audibly performing a predetermined notification based on an instruction of the display control unit 32.

The input unit 7 is an interface for performing a predetermined input to the control unit 3 and can be a keyboard and a mouse.

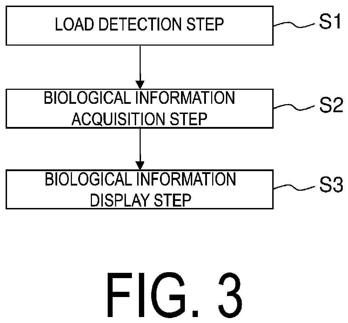

The biological information display system 100 displays the biological information of the subject on the bed according to the steps illustrated in the flowchart of FIG. 3. Specifically, a load detection step S1 for detecting the load of the subject on the bed, a biological information acquisition step S2 for acquiring the biological information of the subject based on the detected load, and a biological information display step S3 for displaying the acquired biological information at the display unit 5 are included.

Each step of the flowchart of FIG. 3 will be described by way of example when the biological information is the respiration rate of the subject on the bed.

Load Detection Step S1

In the load detection step S1, the load detectors 11, 12, 13, and 14 are used to detect the load of the subject S on the bed BD. The load of the subject S on the bed BD are distributed to and detected by the load detectors 11 to 14 located under the legs $BL_1$ to $BL_4$ at the four corners of the bed BD.

Each of the load detectors 11 to 14 detects a load (load change) and outputs the detected load to the A/D conversion unit 2 as an analog signal. The A/D conversion unit 2 converts an analog signal into a digital signal with a sampling period of, for example, 5 milliseconds, and outputs as a digital signal (hereinafter referred to as "load signal", an example of a biological signal) to the control unit 3. Hereinafter, the load signals acquired by digitally converting the analog signals output from the load detectors 11, 12, 13, and 14 at the A/D conversion unit 2 will be referred to as load signals $s_1$, $s_2$, $s_3$, and $s_4$, respectively.

Biological Information Acquisition Step S2

In the biological information acquisition step S2, the biological information acquisition unit 31 calculates (acquires) the respiration rate of the subject S by using the load signals $s_1$ to $s_4$.

The biological information acquisition unit 31 may calculate the respiration rate of the subject S in various ways.

Specifically, for example, the position of a centroid G of the subject S on the bed BD can be calculated based on the load signals $s_1$ to $s_4$, and the respiration rate of the subject S can be calculated based on the temporal variation (trajectory) of the position of the centroid G.

The position of the centroid G is calculated based on the positions of the load detectors 11 to 14 in the coordinates (FIG. 2), and the load signals $s_1$ to $s_4$. The coordinates (FIG. 2) define the width direction and the longitudinal direction of the bed BD to be the X direction and the Y direction, respectively, and define the center of the bed BD to be a center O.

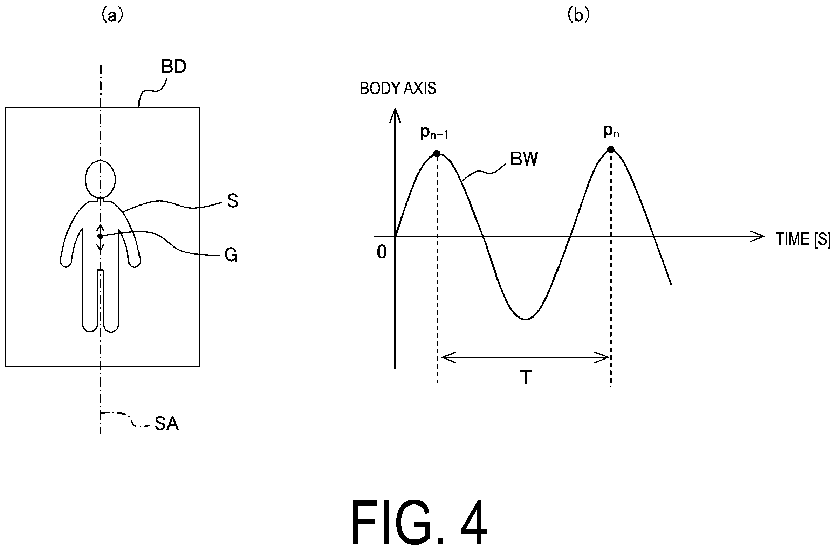

Here, human respiration is performed by moving the thorax and diaphragm to expand and contract the lungs. Thus, as described in the specification of JP 6105703 B granted to the applicant in the present case, the human centroid moves slightly with the movement of the diaphragm and the accompanying movement of internal organs, and the direction of movement is approximately along the direction of extension of the backbone (body axial direction). FIG. 4(a) illustrates how the centroid G of the subject S vibrates in the direction of a body axis SA of the subject S according to the respiration of the subject S.

The biological information acquisition unit 31 acquires a respiration waveform BW (FIG. 4(b)) by plotting, on the vertical axis, the distance between the position of the centroid G projected on the body axis SA at each time and the vibration center of the vibration of the centroid G according to the respiration, with the direction of the body axis SA as the vertical axis and the time axis as the horizontal axis. Then, by using a distance T[s] (equal to the period of respiration) between two consecutive peaks of the respiration waveform BW (peak $p_n$ and peak $p_{n-1}$ in FIG. 4(b)), a calculated value R of the respiration rate per minute is acquired by the following Equation 1.

Equation 1:

$$R = 60 / T$$

In addition, the biological information acquisition unit 31 may determine the respiration rate of the subject S by frequency analysis. Specifically, for example, Fourier analysis is performed on at least one of the load signals $s_1$ to $s_4$ during a predetermined sampling period to identify a peak frequency appearing in a frequency band (human respiration occurs about 12 to 20 times per minute, thereby ranging from about 0.2 Hz to about 0.33 Hz) corresponding to the respiration. The calculated value of the respiration rate of the subject S during the specified period is acquired from the specified peak frequency.

The biological information acquisition unit 31 stores the calculated value of the determined respiration rate at the storage unit 4 together with the time (calculated time) to have calculated the calculated value.

Biological Information Display Step S3

In the biological information display step S3, the display control unit 32 displays the respiration rate of the subject S at the display unit 5 while controlling the content of the information to be displayed.

The display control unit 32 makes the respiration rate displayed at the display unit 5 different as follows between a period (hereinafter referred to as "information acquisition possible period P1", see below for details) when the biological information acquisition unit 31 can acquire the biological information (here, respiration rate) and a period (hereinafter referred to as "information acquisition impossible period P2", see below for details) when the biological information acquisition unit 31 cannot acquire the biological information.

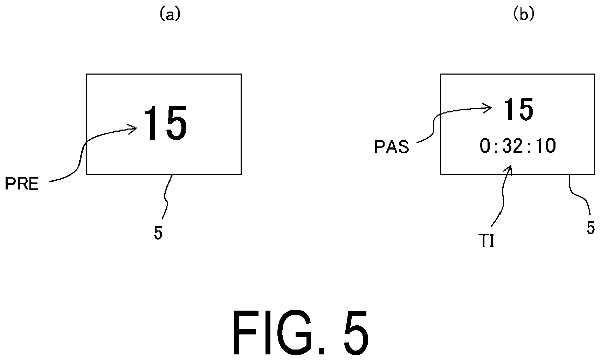

The display control unit 32 displays the latest calculated value of the respiration rate of the subject S stored in the storage unit 4 at the display unit 5 during the information acquisition possible period P1. In this case, the value displayed at the display unit 5 as the respiration rate of the subject S is the present respiration rate (hereinafter referred to as "present respiration rate (present information) PRE") of the subject S (FIG. 5(a)).

On the other hand, the display control unit 32 displays the calculated value of the past respiration rate of the subject S stored in the storage unit 4 at the display unit 5 during the information acquisition impossible period P2. In the present embodiment, the calculated value acquired (stored) last in the immediately preceding information acquisition possible period P1 is used. In this case, the value displayed at the display unit 5 as the respiration rate of the subject S is the past respiration rate (hereinafter referred to as a "past respiration rate (past information) PAS") of the subject S (FIG. 5(b)).

The display control unit 32 also determines the elapsed time from the calculation of the past respiration rate PAS based on the calculated time of the past respiration rate PAS displayed at the display unit 5. Then, the acquired elapsed time (e.g., hours: minutes: seconds) is displayed at the display unit 5 as time information (hereinafter referred to as a "time information TI") indicating the acquired time of the past respiration rate PAS (FIG. 5(b)), together with the past respiration rate PAS. The time information TI is displayed near the character indicating past respiration rate PAS, for example, directly below the character. The displayed elapsed time is updated over time during display.

Thus, by displaying the time information TI together with the past respiration rate PAS, the user of the biological information display system 100 can determine at a glance whether the displayed respiration rate is a present respiration rate PRE or the past respiration rate PAS based on the presence or absence of the display of the time information TI. On the basis of the time information TI, the acquired time of the respiration rate indicated by the past respiration rate PAS can be known.

The information acquisition impossible period P2 (period when the biological information acquisition unit 31 cannot acquire the biological information) includes a period when the biological information acquisition unit 31 cannot acquire the calculated value of the biological information, and a period when the biological information acquisition unit 31 has acquired the calculated value of the biological information but the accuracy of the calculated value is low. In the present embodiment, the calculated value calculated during the information acquisition impossible period P2 is not regarded as biological information.

The display control unit 32 may determine whether a predetermined time is within the information acquisition impossible period P2, by taking into account various factors.

For example, in the present embodiment, the biological information acquisition unit 31 calculates the respiration rate of the subject S based on the variation of the load signals $s_1$ to $s_4$ according to the respiration of the subject S. Thus, the respiration rate (biological information) of the subject S can be acquired with sufficient accuracy during the period when the subject S is present in a rest state on the bed (the period when the subject S is present on the bed and no body movement occurs). Thus, the display control unit 32 may determine that the period when the subject S is not in a rest state (non-rest period) is the information acquisition impossible period P2.

The display control unit 32 may, for example, calculate the total load applied to the bed BD by using the load signals $s_1$ to $s_4$ and determine that the subject S is on the bed BD when the total load is within a predetermined range (e.g., 30 to 150 kg). The display control unit 32 also calculates the standard deviation $\sigma$ indicating the amount of variation in the signal with respect to at least one of the load signals $s_1$ to $s_4$, and when the standard deviation $\sigma$ exceeds a predetermined threshold, and determines that the body movement occurs in the subject S. The display control unit 32 may determine that the subject S is in a rest state when the subject S is resting on the bed BD and the subject S has no body movement, and determine that the other non-rest period is the information acquisition impossible period P2.

The information acquisition impossible period P2 may more specifically include, for example, the following periods. Thus, the display control unit 32 may determine whether the predetermined time is within the information acquisition impossible period P2, taking into consideration whether it is within any of the following periods.

(1) Bed Leaving Period

During a bed leaving period when the subject S is away from the bed BD, the biological information acquisition unit 31 cannot acquire the calculated value of the respiration rate of the subject S because the load signals $s_1$ to $s_4$ reflecting the respiration of the subject S are not input to the biological information acquisition unit 31. Thus, a bed leaving period when the subject S is away from the bed BD is an example of the information acquisition impossible period P2.

The display control unit 32 may, for example, calculate the total load applied to the bed BD by using the load signals $s_1$ to $s_4$, and the case when the total load equals to a predetermined value (as an example, 20 [kg]) or less is determined to be the bed leaving period.

(2) Predetermined Period After System Startup

Immediately after the biological information display system 100 is started up, until the post-startup processing is completed and the system is stabilized, the values of the load signals $s_1$ to $s_4$ and the calculated value of the respiration rate acquired by the biological information acquisition unit 31 are not highly accurate. Accordingly, a predetermined period after the biological information display system 100 is started up is an example of the information acquisition impossible period P2.

The display control unit 32, for example, measures the elapsed time since the startup of the biological information display system 100, and until the measured value becomes a predetermined value (e.g., 30 to 60 [s]) or greater, it may be determined within the predetermined period after the startup of the system.

(3) Predetermined Period After Landing

Immediately after the subject S lands on (enters) the bed BD, the load signals $s_1$ to $s_4$ may fluctuate due to the movement of the subject S or the like, more greatly than the fluctuation caused by the respiration of the subject S. Thus, in the predetermined period after the subject S lands on the bed BD, the accuracy of the calculated value of the respiration rate acquired by the biological information acquisition unit 31 may be lowered due to the effect of the load fluctuation caused by the body movement. Thus, the predetermined period after landing of the subject S on the bed BD is an example of the information acquisition impossible period P2.

The display control unit 32 may, for example, determine that a period until a predetermined time (as an example, 30 to 60 [s]) elapses after the total load applied to the bed BD exceeds a predetermined value (as an example, 20 to 30 [kg]) is within a predetermined period after landing.

(4) Body Movement Occurrence Period

During the period when the subject S has body movement (e.g., rolling over, getting up, or the like), the load signals $s_1$ to $s_4$ may also vary significantly as in the predetermined period after landing. Thus, the accuracy of the calculated value of the respiration rate acquired by the biological information acquisition unit 31 may be lowered due to the effect of the load fluctuation caused by the body movement also during the period when the body movement is occurring in the subject S. Thus, the body movement occurrence period when the body movement is occurring in the subject S is an example of the information acquisition impossible period P2.

(5) When An Error Occurs

Other cases may occur when the respiration rate is not calculated due to some error in the biological state display system 100 (e.g., communication error, or the like). Thus, the error period when the calculated value of the respiration rate is not output from the biological information acquisition unit 31 is an example of the information acquisition impossible period P2.

Specifically, display control by the display control unit 32 is performed as follows. The display control unit 32 periodically determines (e.g., every 1 [s] to 180 [s]) whether the present time is within the information acquisition impossible period P2, and periodically updates the information displayed at the display unit 5 based on the determination result. For example, the display control unit 32 determines to be within the information acquisition impossible period P2, when the subject S is not in the rest state or is in the predetermined period after the system startup, and determines to be within the information acquisition possible period P1 in other cases.

When the display control unit 31 determines that the present time is within the information acquisition impossible period P2, the calculated value that is last acquired in the immediately preceding information acquisition possible period P1 is displayed at the display unit 5 as a past respiration rate PAS, and the elapsed time after the acquisition of the past respiration rate PAS is displayed at the display unit 5 as the time information TI. On the other hand, when the display control unit 32 determines that the present time is within the information acquisition possible period P1, the latest calculated value at the present time is displayed at the display unit 5 as the present respiration rate PRE.

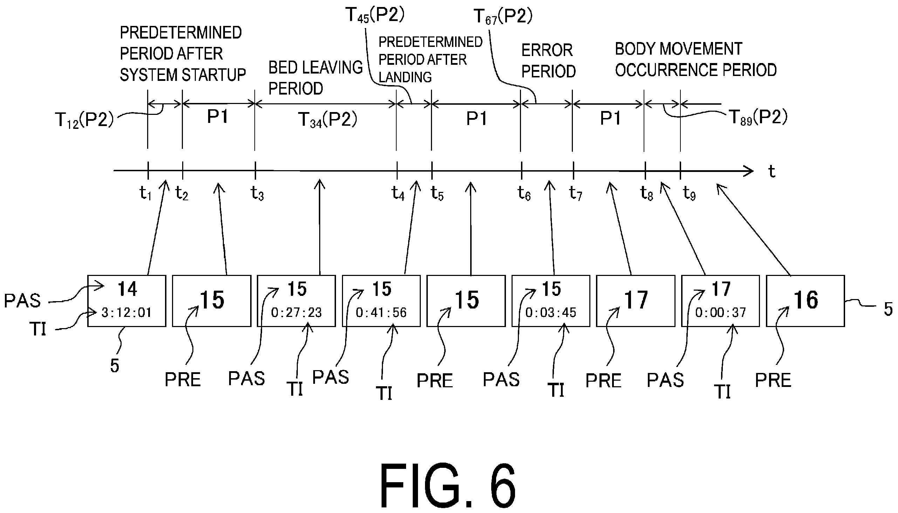

According to the above control, the information displayed at the display unit 5 in each period is illustrated in FIG. 6 as an example.

In a predetermined period $T_{12}$ after system startup, a bed leaving period $T_{34}$, a predetermined period $T_{45}$ after landing, an error period $T_{67}$, and a body movement occurrence period $T_{89}$, the display control unit 32 displays the past respiration rate PAS and the time information TI indicating the elapsed time after the acquisition of the past respiration rate PAS at the display unit 5, based on the determination that the subject S is not in a rest state or the determination that a predetermined time has not elapsed after system startup.

The display control unit 32 displays, for example, in the bed leaving period $T_{34}$ and the predetermined period $T_{45}$ after landing, as the past respiration rate PAS, the respiration rate acquired immediately before the time $t_3$, i.e., last in the preceding information acquisition possible period P1. Similarly, in the error period $T_{67}$ and in the body movement occurrence period $T_{89}$, as the past respiration rate PAS, the last respiration rates acquired last in the information acquisition possible period P1 before the time $t_6$ and the time $t_8$, are displayed respectively.

On the other hand, the display control unit 32 displays the present respiration rate PRE by using the latest calculated value output from the biological information acquisition unit 31 in the information acquisition possible period P1 from the time $t_2$ to the time $t_3$, from the time $t_5$ to the time $t_6$, from the time $t_7$ to the time $t_8$, and after the time $t_9$.

Advantageous effects of the biological information display system 100 of the present embodiments are summarized below.

In the biological information display system 100 of the present embodiments, during the information acquisition impossible period P2, that is, the period when the biological information acquisition unit 31 can acquire the calculated value of the respiration rate and the period when the accuracy of the acquired calculated value is low, the display control unit 32 displays the calculated value of the respiration rate acquired with sufficient accuracy in the past as the past respiration rate PAS at the display unit 5 together with the time information TI indicating the elapsed time from the calculation point. Thus, the display of the biological information at the display unit 5 is not interrupted.

The past respiration rate PAS to be displayed is, although not the latest calculated value, is not the information with accuracy or reliability having been impaired by disturbances or the like. Thus, when this information is presented to the user along with the elapsed time from the calculated time, the user can effectively use the information.

For example, when the user is a doctor or a nurse, the user can determine how to handle the displayed past respiration rate PAS based on the condition of the subject patient (e.g., information described in the medical record, or the like) and the elapsed time from the calculated time of the past respiration rate PAS, and in some cases, the user can determine that the information is equal to the current respiration rate and utilize the past respiration rate PAS.

Thus, even during a period when the current respiration rate cannot be acquired with sufficient accuracy, the biological information display system 100 of the present embodiments can display, to the user, useful biological information such as the respiration rate acquired with sufficient accuracy in the past and the elapsed time from the acquisition time of the respiration rate.

Modifications

In the biological state display system 100 of the above embodiments, the following modification mode may be also adopted.

In the above embodiments, the elapsed time from the time when the past respiration rate PAS is calculated is displayed as the time information TI. However, the time information TI is not limited to this, and can be any information indicating the calculated time (acquired time) of the past respiration rate PAS.

Specifically, for example, the time and/or date when the displayed past respiration rate PAS is calculated may be displayed as the time information TI.

In addition, the display mode of character information such as the elapsed time and calculated time displayed as the time information TI may be changed step by step over time. Specifically, for example, the color of a character indicating the elapsed time or calculated time is changed in such modes: blue until one hour has elapsed, green after one hour has elapsed, red after three hours have elapsed, etc. Thus, the user can intuitively understand how old the displayed past respiration rate PAS is based on the color of characters indicating the elapsed time or the calculated time.

The display mode changing over time is not limited to color. For example, the font and size of the characters, the presence or absence of shading on the background of the characters, the design of the shading, the presence or absence of an enclosure around the characters, the design of the enclosure, or the like may be changed over time.

Additionally, the time information TI is not limited to character information. For example, a color bar, an icon, or the like may be used to change the color of the color bar and the design of the icon over time. In this case, the user can intuitively understand the calculated time of the displayed past respiration rate PAS based on the color of the color bar and the design of the icon.

The time information TI does not necessarily need to be displayed separately from the past respiration rate PAS around the character information indicating the past respiration rate PAS. For example, when the time information is displayed by the color bar, the color bar may be displayed behind character information (numbers) indicating the past respiration rate PAS.

Alternatively, the time information TI may be displayed by changing the display mode of the past respiration rate PAS itself over time. Specifically, for example, the color of the characters (numbers) indicating the past respiration rate PAS is changed to blue, green, red, or the like over time. In this case, the past respiration rate (past information) PAS is displayed by a value indicated by the number (character), and the time information TI is displayed by the color of the number (character). The display mode to be changed over time is not limited to the color, but can be the font and size of the characters, the presence or absence of shading on the background of the characters, the design of the shading, the presence or absence of an enclosure around the characters, the design of the enclosure, and the like. Thus, the past respiration rate PAS and the time information TI can be displayed compactly, making the display unit 5 smaller.

In addition, the past respiration rate PAS and the time information TI may be displayed in various ways, and the above modifications may be combined appropriately.

In the biological information display system 100 of the above embodiments, the calculated value of the respiration rate acquired last during the immediately preceding information acquisition possible period P1 is set as the past respiration rate PAS displayed during the information acquisition impossible period P2, but not limited to this setting. The calculated value (biological information) acquired at any timing of any past information acquisition possible period P1 can be used as the past information to be displayed during the information acquisition impossible period P2.

In the above embodiments, the case of displaying the respiration rate of the subject S as the biological information has been described by way of example, but not limited to this example. The biological information may be any biological information such as heart rate in addition to respiration rate. The biological information is not limited to be displayed numerically, but may be displayed as a graph of a time series, for example. In this case, the above description applies as well.

For example, in the case of displaying the heart rate, the biological information acquisition unit 31 performs Fourier analysis on at least one of the load signals $s_1$ to $s_4$ during a predetermined sampling period and identifies the peak frequency appearing in the frequency band corresponding to the heart rate (from about 0.5 Hz to about 3.3 Hz). The calculated value of the heart rate of the subject S during the period is acquired from the specified peak frequency.

In the above embodiments, determination as to whether the information acquisition impossible period P2 is determined by the display control unit 32 can be made in various modes. For example, in addition to or instead of determining whether the subject S is in a rest state, at least one of the following may be used: determining whether during a bed leaving period; determining whether during a predetermined period after system startup; determining whether during a predetermined period after landing; determining whether during a body movement occurrence period; and determining whether during an error period. The accuracy of the determination can be improved by combining many determinations.

The display control unit 32 need not consider all of the non-rest period, bed leaving period, predetermined period after system startup, predetermined period after landing, body movement occurrence period, and error period as the information acquisition impossible period P2. The display control unit 32 may consider at least one of the non-rest period, bed leaving period, predetermined period after system startup, predetermined period after landing, body movement occurrence period, and error period as the information acquisition impossible period P2 and display the past respiration rate PAS during the period.

When the biological information to be displayed at the display unit 5 becomes a predetermined value, the display control unit 32 may use the notification unit 6 to provide a voice notification. The predetermined value is entered and set, for example, by the user of the biological information display system 100 via the input unit 7.

In the biological information display system 100 of the above embodiments, any biological signal acquisition unit for acquiring the biological signal of the subject S (a signal fluctuating with the biological activity of the subject S) can be used instead of the load detection unit 1. Specifically, for example, a plurality of pressure sensors (pressure sensors) arranged in a matrix form under a bed sheet can acquire a variation in the pressure applied by the subject S to the bed BD as a biological signal. In this mode, the position of the centroid of the subject, the respiration rate, the heart rate, or the like can be determined based on the output of the plurality of pressure sensors.

The biological information display system 100 of the above embodiments need not necessarily include all of the load detectors 11 to 14, but may only include one of these detectors. For example, when three load detectors are used, the position of the centroid of the subject S on the bed BD plane can be detected as long as the load detectors are not arranged in a straight line. Furthermore, the load detectors do not necessarily need to be located at the four corners of the bed, but can be located at any location so that the load and the variation of the load of the subject on the bed can be detected. As the load detectors 11 to 14, for example, a force sensor can be used without limited to a load sensor using a beam-type load cell.

In the biological information display system 100 of the above embodiments, the display unit 5 may be provided with a printer for printing and outputting information indicating a biological state (respiration rate, presence or absence of body movement, or the like) or a simple visual display means such as a lamp for indicating a biological state (respiration rate, presence or absence of the body movement, or the like) instead of or in addition to a monitor. The notification unit 6 may be provided with a vibration generating unit for notifying by vibration instead of or in addition to a speaker.

To the extent that the features of the present invention are maintained, the present invention is not limited to the above embodiments, and other forms conceivable within the scope of the technical concept of the present invention are also included within the scope of the present invention.

Reference Signs List

1 Load detection unit; 11,12,13,14 Load detector; 2 A/D conversion unit; 3 Control unit; 31 Biological information acquisition unit; 32 Display control unit; 4 Storage unit; 5 Display unit; 6 Notification unit; 7 Input unit; 100 Biological state display system; BD Bed; S Subject

**Claims**

1. A biological information display system for displaying biological information of a subject on a bed, the biological information display system comprising:

   a biological signal acquisition unit configured to acquire a biological signal of the subj ect;
   a biological information acquisition unit configured to acquire biological information of the subject based on the biological signal; and
   a display control unit configured to display the biological information at a display unit, wherein during a period when the biological information acquisition unit cannot acquire the biological information, the display control unit displays past information being the biological information acquired before the period at the display unit together with time information indicating a time when the past information is acquired.

2. The biological information display system according to claim 1, wherein
the display control unit changes a display mode of the time information according to an elapsed time from the time when the time information is acquired.

3. The biological information display system according to claim 1, wherein
the display control unit displays the time information by changing a display mode of the past information.

4. The biological information display system according to any one of claims 1 to 3, wherein
the time information is an elapsed time from the time when the past information is acquired.

5. The biological information display system according to claim 1 or 2, wherein
the time information is a date and/or time when the past information is acquired.

6. The biological information display system according to any one of claims 1 to 5, wherein
the period when the biological information acquisition unit cannot acquire the biological information includes at least one of a non-rest period, a bed leaving period when the subject is not present on the bed, a predetermined period after startup of the biological information display system, a predetermined period after the subject lands on the bed, a period when body movement is occurring in the subject, and an error period.

7. The biological information display system according to any one of claims 1 to 6, wherein
the biological signal acquisition unit includes a load detector configured to acquire, as a biological signal of the subject, a load signal varying according to respiration and/or heartbeat of the subject.

FIG. 1

EP 4 378 376 A1

# FIG. 2

```
┌─────────────────────────────┐
│      LOAD DETECTION STEP     │ ⌇ S1
└─────────────────────────────┘
              │
              ▼
┌─────────────────────────────┐
│   BIOLOGICAL INFORMATION     │ ⌇ S2
│      ACQUISITION STEP        │
└─────────────────────────────┘
              │
              ▼
┌─────────────────────────────┐
│   BIOLOGICAL INFORMATION     │ ⌇ S3
│        DISPLAY STEP          │
└─────────────────────────────┘
```

# FIG. 3

(a)

(b)

FIG. 4

(a)

(b)

FIG. 5

FIG. 6

EP 4 378 376 A1

## INTERNATIONAL SEARCH REPORT

| International application No. |
|---|
| **PCT/JP2022/028495** |

**A. CLASSIFICATION OF SUBJECT MATTER**

*A61B 5/00*(2006.01)i; *A61B 5/11*(2006.01)i; *A61B 5/113*(2006.01)i
FI: A61B5/00 D; A61B5/113; A61B5/11 100; A61B5/00 102E

According to International Patent Classification (IPC) or to both national classification and IPC

**B. FIELDS SEARCHED**

Minimum documentation searched (classification system followed by classification symbols)

A61B5/00; A61B5/11; A61B5/113

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2018/097133 A1 (PARAMOUNT BED CO., LTD.) 31 May 2018 (2018-05-31) paragraphs [0004]-[0005], [0010], [0015]-[0019], [0028], [0057], fig. 1, 4 | 1-7 |

☐ Further documents are listed in the continuation of Box C.       ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **17 August 2022** | **30 August 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT

Information on patent family members

International application No.

**PCT/JP2022/028495**

| Patent document cited in search report | Publication date (day/month/year) | Patent family member(s) | Publication date (day/month/year) |
|---|---|---|---|
| WO 2018/097133 A1 | 31 May 2018 | US 2019/0159738 A1<br>paragraphs [0005]-[0006], [0011], [0027]-[0031], [0043], [0077], fig. 1, 4<br>US 2022/0133243 A1<br>CN 109952616 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6697985 B
- JP 4829020 B
- JP 4002905 B
- JP 6105703 B